# EUROPEAN PATENT SPECIFICATION

(11) **EP 2 528 571 B1**
(45) Date of publication and mention of the grant of the patent: **12.10.2016**
(21) Application number: 11703955.2
(22) Date of filing: 26.01.2011
(51) Int. Cl.: A61F 13/20, A61F 13/34, A61L 15/16

(54) **TAMPON HAVING A WITHDRAWAL STRING COMPRISING A FLUOROCARBON COMPOUND**
TAMPON MIT EINER ENTNAHMESCHNUR MIT EINER FLUORKOHLENSTOFFVERBINDUNG
TAMPON COMPORTANT UN CORDON DE RETRAIT COMPRENANT UN COMPOSÉ DE FLUOROCARBONE

(30) Priority: 27.01.2010 US 694608
(43) Date of publication of application: 05.12.2012
(73) Proprietor: The Procter & Gamble Company, Cincinnati, OH 45202 (US)
(72) Inventor: MINOGUCHI, Ryo, Cincinnati, Ohio 45242 (US); ARORA, Kelyn, Anne, Cincinnati, Ohio 45226 (US); PEACE, William, Patton, Lawrenceburg, Indiana 47025 (US)
(74) Representative: Kremer, Véronique Marie Joséphine
(86) International application number: PCT/US2011/022479
(87) International publication number: WO 2011/094239

(56) References cited:
- EP-A1- 1 481 656
- WO-A1-97/14386
- US-A- 5 458 589
- US-A1- 2002 095 128

## Description

### FIELD OF THE INVENTION

The invention relates to tampons having an improved withdrawal string, and more particularly to tampons having an improved withdrawal string comprising one or more fluorocarbon compounds.

### BACKGROUND OF THE INVENTION

Feminine hygiene devices, such as tampons and pessaries, are generally used by women within the vagina for feminine needs, such as, *e.g.,* to absorb menstrual or other body exudates and/or for pelvic support. Typically, feminine hygiene devices can be inserted by using an applicator or digitally with a finger. In addition, many of these devices include a withdrawal string to facilitate removal of these devices after use. Such withdrawal strings are typically twisted or knitted plies of yarns composed of cellulosic fibers such as cotton. Because the withdrawal string is positioned within the vagina and labial space during use of the device, however, the string may contact body fluids such as menses or urine and become soiled and wet. This can lead to an unpleasant removal experience.

US2002/095128 relates to a withdrawal string for use with an absorbent pledget. The withdrawal string is affixed to an absorbent pledget which is insertable into a body cavity. The withdrawal string is a multifilamentous twine having a fixed end attached to the pledget and a free end. At least one barrier portion is disposed between the free end and the fixed end of the withdrawal string. The barrier portion may be formed by heating, crimping, solvent fusion or, most preferably, by permeating one or more segments of the withdrawal string between the free end and the pledget with an uncured elastomer and curing the elastomer to provide a viscous diffusion barrier.

As such, it would be desirable to provide a feminine hygiene device having an improved withdrawal string. It would also be desirable to provide a feminine hygiene device having an improved withdrawal string that exhibits reduced adhesion and/or wicking of fluid.

### SUMMARY OF THE INVENTION

Feminine hygiene devices having a withdrawal string comprising one or more fluorocarbon compounds are provided.

In certain embodiments, the feminine hygiene device can include a body having an insertion end, a withdrawal end, and a longitudinal axis. The feminine hygiene device can also have a withdrawal string extending from the withdrawal end, the withdrawal string comprising one or more fluorocarbon compounds.

In certain embodiments, the feminine hygiene device can include a body having an insertion end, a withdrawal end, and a longitudinal axis. The feminine hygiene device can also have a withdrawal string extending from the withdrawal end, the withdrawal string comprising one or more scoured synthetic fibers, the scoured fibers comprising one or more fluorocarbon compounds.

Methods of making a feminine hygiene device having a withdrawal string comprising one or more fluorocarbon compounds are also provided.

### BRIEF DESCRIPTION OF THE DRAWINGS

FIG. 1 is a perspective view of one embodiment of the present invention.
FIG. 2 is a perspective view of one embodiment of the present invention.

### DETAILED DESCRIPTION OF THE INVENTION

The present invention relates to feminine hygiene devices that include a withdrawal string comprising one or more fluorocarbon compounds. In certain embodiments, the one or more fluorocarbon compounds can be resin-incorporated. In addition, in certain embodiments, the withdrawal string can comprise one or more one or more scoured fibers, the scoured fibers comprising one or more fluorocarbon compounds. Such withdrawal strings can demonstrate an improved tampon string having reduced or eliminated wicking and adhesion of fluid such as, *e.g.,* menses.

As used herein, the term "feminine hygiene device" includes absorbent articles useful for feminine needs, such as articles that typically can be intended for feminine use internally, such as, *e.g.,* within a user's vagina. Internal feminine hygiene devices can include, for example, tampons and pessaries.

As used herein, the term "tampon" refers to any type of absorbent structure that can be inserted into the vaginal canal or other body cavity, such as, *e.g.,* for the absorption of fluid, to aid in wound healing, and/or for the delivery of materials, such as moisture or active materials such as medicaments.

As used herein, the term "pessary" refers to any type of substantially non-absorbent structure for the purpose of reducing urine leakage and/or supporting a prolapsed uterus and/or bladder. Such pessaries can have any variety of shapes and sizes including cylinder, ovate, spherical, tubular, annual rings, "U" shaped, cup shaped, rings, cubes or donut shaped, and can function in any suitable manner, such as, *e.g.,* by direct application of support, lever force, expansion of the device by selection of material, and/or by inflation of the device.

As used herein, the term "vaginal canal" refers to the internal genitalia of the human female in the pudendal region of the body. The terms "vaginal canal" or "within the vagina" as used herein are intended to refer to the space located between the introitus of the vagina (sometimes referred to as the sphincter of the vagina) and the cervix.

As used herein, "applicator" refers to a device or implement that facilitates the insertion of a feminine hygiene device, such as, *e.g.,* a tampon or pessary, into an external orifice of a mammal. Exemplary applicators include telescoping, tube and plunger, and compact applicators.

The term "joined" or "attached" as used herein, encompasses configurations in which a first element is directly secured to a second element by affixing the first element directly to the second element, configurations in which the first element is indirectly secured to the second element by affixing the first element to intermediate member(s) which in turn are affixed to the second element, and configurations in which first element is integral with second element, i.e., first element is essentially part of the second element.

As used herein, the term "scoured" refers to removal of a finish composition. With the synthetic fibers of the invention, removal of the spin finish can create a hydrophobic yarn that is substantially finish free, such as, e.g., having less than about 80% finish composition, about 70% finish composition, about 60% finish composition, about 50% finish composition, about 40% finish composition, about 30% finish composition, about 20% finish composition, about 10% finish composition, or substantially no finish composition.

As used herein, the term "synthetic fiber" includes non-natural and substantially nonabsorbent fibers, such as, for example, polyethylene fibers, polypropylene fibers, polyethylene-polypropylene copolymer fibers, polyvinyl alcohol fibers, polyvinyl acetate fibers, polyester fibers, nylon fibers, polylactide fibers, polyhydroxylalkanoate fibers, aliphatic ester polycondensate fibers, cellulose acetate fibers, and mixtures thereof. In certain embodiments, the synthetic fiber can include a fluoropolymer, such as, for example, a fluoroolefin. Rayon fibers are not considered synthetic fibers as used herein. In certain embodiments, the synthetic fiber can be a bicomponent or multicomponent fiber.

As used herein, the term "multicomponent fiber" refers to fibers that have been formed from at least two component polymers, or the same polymer with different properties or additives, extruded from separate extruders but spun together to form one fiber. Multicomponent fibers are also sometimes referred to as conjugate fibers or bicomponent fibers, although more than two components may be used. The polymers are arranged in substantially constantly positioned distinct zones across the cross-section of the multicomponent fibers and extend continuously along the length of the multicomponent fibers. The configuration of such a multicomponent fiber may be, for example, a sheath/core arrangement wherein one polymer is surrounded by another, or may be a side by side arrangement, an "islands-in-the-sea" arrangement, or arranged as pie-wedge shapes or as stripes on a round, oval or rectangular cross-section fiber, or other. For two component fibers, the polymers may be present in ratios of 75/25, 50/50, 25/75 or any other desired ratios. In addition, any given component of a multicomponent fiber can comprise two or more polymers as a multiconstituent blend component.

Feminine hygiene devices having a withdrawal string comprising one or more scoured synthetic fibers are provided.

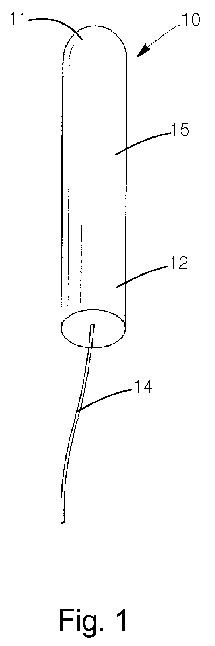

Figure 1 shows one embodiment of an absorbent tampon 10 of the present invention. The tampon 10 can include a body 15 having an insertion end 11 and a withdrawal end 12. The tampon 10 also includes a withdrawal string 14 joined to the body 15 at withdrawal end 12. As shown in Figure 1, the withdrawal string 14 can be directly joined to the body 15.

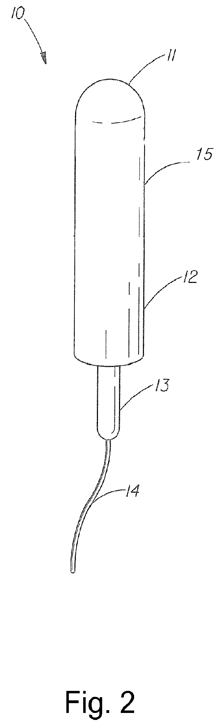

Figure 2 shows one embodiment of an absorbent tampon 10. The tampon 10 can include a body 15 having an insertion end 11 and a withdrawal end 12. The tampon 10 also includes a withdrawal string 14 joined to the body 15 at withdrawal end 12. In certain embodiments, the tampon 10 can include a secondary absorbent 13. As shown in Figure 2, in certain embodiments, the secondary absorbent can extend from the withdrawal end 12 and the withdrawal string 14 can be directly joined to the secondary absorbent 13.

The withdrawal string can comprise any suitable synthetic material. Suitable synthetic materials include, for example, non-natural material, such as, for example, polyethylene, polypropylene, polyethylene-polypropylene copolymer, polyvinyl alcohol, polyvinyl acetate, polyester, nylon, polylactides, polyhydroxylalkanoates, aliphatic ester polycondensates, cellulose acetate, and mixtures thereof. In certain embodiments, the synthetic material can be a fluoropolymer, such as, for example, a fluoroolefin, such as, for example, a fluoropolypropylene. In certain embodiments, the synthetic material includes a polymer composition that includes homopolymers, copolymers, such as, for example, block, graft, random and alternating copolymers, terpolymers, etc., and blends and modifications thereof. Any suitable geometrical configurations of the material can be employed, such as, for example, isotactic, syndiotactic and random symmetries.

The withdrawal string can be formed using synthetic fibers. Such fibers can be formed in any suitable manner, such as, e.g., by melt spinning to produce staple fibers, monofilaments, multifilaments, continuous filaments, or other fibers useful in forming a withdrawal string. Fiber formation and processing generally includes the addition of finish compositions, such as, for example, lubricants and anti-statics. In certain embodiments, the fiber can be a bicomponent or multicomponent fiber.

The withdrawal string includes one or more suitable fluorocarbon compounds. The one or more suitable fluorocarbon compounds include fluoropolymers such as polytetrafluoroethylene (PTFE), perfluoroalkoxy (PFA), fluorinated ethylene propylene (FEP), polyvinyl fluoride (PVF), polyfluorovinylidene (PVDF), fluoroolefins, chlorofluorocarbons, hydrofluorocarbons, fluorosurfactants, and derivatives. Suitable fluorosurfactants include, for example, Zonyl (DuPont), Capstone (DuPont), MaSurf FS Fluorosurfactants (Mason Chemical Company), or PolyFox Fluorosurfactants (Onmova). In certain embodiments, the withdrawal string is formed from a synthetic material, such as, for example, polypropylene, and a fluorocarbon compound is added to the synthetic material, such as, for example, by resin-incorporation of the fluorocarbon compound into the synthetic material or by coating the synthetic material with the fluorocarbon compound. Alternatively, in certain embodiments, the withdrawal string is formed from a synthetic fluorocarbon polymer, such as, for example, a fluoroolefin.

The fluorocarbon compounds can be incorporated into the withdrawal string in any suitable amount, such as, for example, greater than about 0.5%, about 1%, from about 1% to about 10%, about 1% to about 8%, about 1% to about 5%, or about 1 to about 3 %.

Fluorocarbon compounds can be incorporated into the withdrawal string in any suitable manner, such as, for example, by resin-incorporation, by coating, or by any other suitable manner. In certain embodiments, the fluorocarbon compound can be incorporated throughout the withdrawal string. Alternatively, the fluorocarbon compound can be provided in a portion of the withdrawal string, such as, for example, in one or more yarns of the string, or in one or more components of a bicomponent or multicomponent fiber. In certain embodiments, the withdrawal string can comprise bicomponent and/or multicomponent fibers, and the fluorocarbon compound can be incorporated into only one component, such as, for example, a sheath component of a sheath/core bicomponent fiber.

In certain embodiments, the withdrawal string can comprise scoured fibers. The finish can be scoured from the fibers, filament, yarn, or string, by any suitable method, such as, *e.g.,* by rinsing the starting fiber, the intermediate yarn, or the finished string with water, water with a detergent, water with a surfactant, supercritical CO₂, or any other solvent suitable for a specific type of the finish to be removed, followed by drying. In certain embodiments, the finish can be scoured at an elevated temperature, such as, for example, at temperatures of about 50°C, about 75° C, or any other suitable temperature, such as, for example, temperatures between about 40°C and about 100°C, such as, for example, temperatures between about 50°C and about 75°C.

The withdrawal string can be formed by any suitable string formation method and in any suitable configuration, such as, e.g., one or more cords, strings, finger covers, ribbons, an extension of a material of the device, or combinations thereof. In certain embodiments, the withdrawal string can include any suitable number of plies of yarn, such as, for example, 1, 2, 3, 4, 5, 6, 7, 8, 9, 10 or more plies of yarn. In certain embodiments, the string can be heat treated, such as, for example, using a heat lamp or other suitable heating device. In certain embodiments, the string can be heat treated to provide a smoother surface as compared to a string that has not been heat treated.

The withdrawal string can be joined to any suitable location on the tampon. As shown in Figures 1-2, the withdrawal string can be joined to the tampon body either directly or indirectly and can extend freely beyond the withdrawal end of the tampon body. The withdrawal string can be attached to the tampon body in any suitable manner, such as, e.g., by stitching, adhesive, or any other suitable manner. In certain embodiments, the withdrawal string can be stitched onto the tampon pledget according to the stitching manner called "Double Ring Stitching" described in Japanese Industrial Standards (JIS) No. B 9070.

The withdrawal string can optionally be provided with a secondary absorbent member. Suitable secondary absorbent members are described in, e.g., U.S. Patent No. 6,258,075. In certain embodiments, the secondary absorbent member can be a material that is the same or different than the withdrawal string. In addition, or alternatively, the secondary absorbent member does not comprise the fluorocarbon compound and/or is not scoured. In certain embodiments, the secondary absorbent member can be colored, such as, purple, blue, violet, green, yellow, orange, red, pink, or declinations thereof. In certain embodiments, the secondary absorbent can be a darker color than the withdrawal string, such as, for example, a blue, purple, violet, or green secondary absorbent joined to a white or other light colored withdrawal string.

The withdrawal string can be any suitable construction. For example, in certain embodiments, the withdrawal string can be a twisted or knitted construction containing multiple plies of yarn. The plies of yarn can be formed from twisted staple fibers or multiple continuous filaments. In addition, or alternatively, the weight or count of the yarn and/or the number of plies can be any weight or plies suitable for providing the desired strength, aesthetics, grippability, wearing comfort, handling, cost, and/or processing. In certain embodiments, the withdrawal string can be formed from 6 twisted plies of 10 count polypropylene yarn, one knitted ply of 10 count polypropylene yarns, or any other suitable construction

The fibers can have any suitable cross-sectional shape, such as, e.g., round, tri-lobal, multi-lobal, delta, hollow, ribbon-shaped, and/or any other suitable shape, or mixtures thereof. Fibers with any suitable diameter can be used, such as, e.g., from about 0.5 to about 50 microns, such as, e.g., from about 1 to about 30 microns, such as, e.g., from about 10 to about 25 microns. Fiber diameter can be determined using any suitable means; however, for non-round fibers, diameter can typically be determined by reference to the diameter of a fiber with the same cross-sectional area as the non-round fiber.

In certain embodiments, the fiber can be a multicomponent fiber. Suitable multicomponent fibers can include, for example, multicomponent fibers that have more than one separate part in spatial relationship to one another, such as, for example, a bicomponent fiber. The different components of multicomponent fibers are arranged in substantially distinct regions across the cross-section of the fiber and extend continuously along the length of the fiber. In certain embodiments, the multicomponent fiber can be a bicomponent fiber. Suitable bicomponent fibers can include, for example, bicomponent fibers that can comprise first and second polymeric components that are coextruded so as to provide the fiber with certain desirable properties from each of the polymeric components. The bicomponent fiber can be of any suitable configuration. Exemplary configurations include, for example, sheath-core, island-in-the-sea, side-by-side, segmented pie, and combinations thereof.

The withdrawal string can optionally include one or more suitable additional ingredients. Suitable additional ingredients include, but are not limited to, those which are typically used in fiber making, such as, for example, nucleating agents, antiblock agents, antistatic agents, a different polymer, pro-heat stabilizers, softening agents, lubricants, surfactants, wetting agents, plasticizers, light stabilizers, weathering stabilizers, weld strength improvers, slip agents, dyes, antioxidants, flame retardants, pro-oxidant additives, natural oils, synthetic oils, anti-blocking agents, fillers, coefficient of friction modifiers, humectants, and combinations thereof. In certain embodiments, the withdrawal string can include one or more additives and/or coatings, such as, e.g., wax, silicones, and/or siloxanes.

Any additional ingredients can be utilized at an amount effective to achieve the result the additional ingredient is present in the polymeric mixture to achieve. For example, a stabilizing amount for a UV stabilizer, a lubricating amount for a lubricating agent. For a skin conditioning agent, an amount of the agent that has an effect on the skin would be desired. In certain embodiments, the additional ingredient can be from about 0.1% to about 5% of the composition. These additional ingredients may be employed in conventional amounts although, typically, such ingredients are not required in the composition in order to obtain the advantageous reduction in wicking.

The tampon can be formed in any suitable manner. In certain embodiments, absorbent material can be joined to an overwrap. In addition, or alternatively, the absorbent member and/or overwrap can be rolled and/or folded, compressed and optionally heat conditioned in any suitable conventional manner to form the tampon. In certain embodiments, after rolling or folding and compression, the overwrap can cover the exterior surface of the compressed absorbent member and can also be embedded in the interior folds of the compressed absorbent member. That is, in certain embodiments, the overwrap can permeate the interior of the compressed absorbent member.

The absorbent material can be any suitable shape, size, material, or construction prior to compression and/or shaping. For example, the pledget can include a rolled, tubed, or flat construction of an absorbent that can be a circle, an oval, a semi-circle, a triangle, a chevron shape, an H shape, a bow-tie shape, or any other suitable shape, such as, e.g., shapes described in, for example, U.S. Pat. Nos. 3,738,364; 5,911,712; 6,740,070; 6,887,266; and 6,953,456. A typical size for absorbent material prior to compression can be from about 30 mm to about 100 mm in length and from about 30 mm to about 80 mm in width. The typical range for the overall basis weight of the absorbent material 28 is from about 150 gsm to about 1250 gsm depending upon desired absorbent capacity. The materials for the tampon can be formed into a fabric, web, or batt that is suitable for use in the absorbent material by any suitable process such as airlaying, carding, wetlaying, hydroentangling, needling or other suitable techniques. In certain embodiments, the absorbent material can be a single pledget that can be compressed to form a tampon.

The absorbent material can be a laminar structure comprised of integral or discrete layers. In other embodiments, the pad need not have a layered structure at all. The absorbent material may comprise a folded structure or may be rolled. The resulting compressed absorbent member of the tampon can be constructed from a wide variety of liquid-absorbing materials commonly used in absorbent articles. Such materials include, for example, rayon (such as GALAXY rayon (a tri-lobed rayon) or DANUFIL rayon (a round rayon), both available from Kelheim Fibres GmbH of Kelheim, Germany), cotton, folded tissues, woven materials, nonwoven webs, synthetic and/or natural fibers or sheeting, comminuted wood pulp, which is generally referred to as airfelt, foams, or combinations of these materials. Examples of other suitable materials include: creped cellulose wadding; meltblown polymers including coform; chemically stiffened, modified or cross-linked cellulosic fibers; synthetic fibers such as crimped polyester fibers; peat moss; foam; tissue including tissue wraps and tissue laminates; or any equivalent material or combinations of materials, or mixtures of these. Additionally, superabsorbent materials, such as superabsorbent polymers or absorbent gelling materials can be incorporated into the tampon.

Any suitable pressures and temperatures for compression can be used. In certain embodiments, the absorbent material and the overwrap can be compressed in the radial direction and optionally axially by any suitable means. While a variety of techniques are known and acceptable for these purposes, a tampon compressor machine available from Hauni Machines, Richmond, Va., can be suitable.

As set forth herein, in certain embodiments, the tampon can be a tampon having a folded construction. Alternatively, the tampon can be a tampon having a radially compressed rolled construction. The tampon can be constructed by rolling and radially compressing the pledget. The pledget can be rolled around a mandrel then compressed with or without the mandrel. In certain embodiments, a cavity left behind after the mandrel is removed can provide a finger pocket. The tampon can also be constructed by pressing a pledget, such as, for example, a cylindrical pledget, in forming dies with a pushrod. A cavity that can be a finger pocket can be formed in part of the blank pressed against a convex in the forming dies or the pushrod.

### Example 1:

This example demonstrates the adhesion of sheep blood to withdrawal strings comprising a fluorochemical compound.

Continuous filament samples were obtained from FiberVisions Corporation (Covington, GA). Filaments comprising polypropylene resin with no additional additives were prepared as a control sample. Filaments comprising polypropylene with different types and amounts of resin-incorporated fluorochemical additives were also prepared to determine the effect on the adhesion. Before testing, each of the filament bundles was scoured to remove spin finishes by soaking in hot water for at least 10 minutes, then removed, rinsed with fresh water under the tap for at least 1 minute and then placed on paper towels to dry.

The filament samples were cut into 2 cm long samples. The filaments were cut to produce a clean and perpendicular cut across the shaft of the filament. The straight samples were placed vertically into a tensiometer (Type K100) available from Kruss GmbH, Hamburg, Germany and the fluid cup in the tensiometer was filled with defibrinated sheep blood (Cleveland Scientific, Ltd., Bath, Ohio). All measurements were taken at a room temperature of 21.2° C and a humidity of approximately 20%. The samples were hung down from the sample holder such that the end cut was perpendicular to the sheep blood. The tensiometer was programmed to raise the vessel at 6 mm/min until the sample made contact with the surface, at approximately 1 mm immersion. If the sample bent while entering the fluid, the run was rejected. Surface detection sensitivity was 0.0001 g, thus when the sample gained 0.0001 g, the instrument recorded that position as the sample making contact with the surface. The tensiometer was programmed to immerse the sample by raising the vessel at 3 nm/min in 0.2 mm increments, waiting 5 seconds at each increment to take a measurement of force on the sample. The sample was immersed 5 mm and then retracted 4 mm. The sample was then immersed 4 mm and retracted 4 mm, and immersed 4 mm and retracted 4 mm for a total of 3 immersions per sample. All movements were at a 3 mm/min speed in 0.2 mm increments. All measurements were automated and the last run was measured to achieve the final measurements as shown in Table 1. The measured force represents adhesion between the filament sample and the sheep blood, and thus lower force represents less adhesion with the fluid.
Table 1 summarizes the filament samples prepared and the resultant adhesion forces.

**Table 1:**

| Sample Number | Additive Type | Additive Concentration | Adhesion Force |
|---|---|---|---|
| TPL#09-1074-06 | none | n/a | 0.37 dyne |
| TPL#09-1119-01 | Fluoro-surfactant masterbatch* | 3% | 0.13 dyne |
| TPL#09-1119-03 | Fluoro-surfactant masterbatch* | 7% | - 0.94 dyne |
| TPL#09-1136-01 | Teflon PTFE | 0.5% | - 0.05 dyne |
| TPL#09-1136-02 | Teflon PTFE | 1.0% | - 0.24 dyne |
| TPL#09-1136-03 | Teflon PTFE | 2.0% | - 0.44 dyne |

| | | | |
|---|---|---|---|
| * Fluoro-surfactant additive masterbatch was obtained from Ampacet Corporation (Tarrytown, NY) as product number LR-170391, "Surface Modified PP Masterbatch" | | | |

As shown in this Example, filament samples including a fluorocarbon compound demonstrate reduced adhesion with the sheep blood compared to filament samples without the fluorocarbon compound.

## Claims

1. A feminine hygiene device comprising: a body having an insertion end, a withdrawal end, a longitudinal axis, the feminine hygiene device comprising a withdrawal string extending from the withdrawal end, the withdrawal string comprising one or more fluorocarbon compounds.

2. The feminine hygiene device of claim 1, wherein the withdrawal string comprises two or more plies of yarn and/or one or more knitted plies of yarn.

3. The feminine hygiene device of claim 1 or 2, wherein the withdrawal string comprises synthetic fibers, and the synthetic fibers are polypropylene and/or fluoropolypropylene.

4. The feminine hygiene device of claim 3, wherein the synthetic fibers are scoured synthetic fibers.

5. The feminine hygiene device of any of claims 1-4, wherein the fluorocarbon compound is resin-incorporated into the fibers and/or is coated onto the fibers.

6. The feminine hygiene device of any of claims 1-5, wherein the fluorochemical compound is polytetrafluoroethylene, perfluoroalkoxy, fluorinated ethylene propylene, polyvinyl fluoride, polyfluorovinylidene, a fluorosurfactant, or a derivative.

7. The feminine hygiene device of any of claims 1-6, wherein the device comprises a secondary absorbent attached to the compressed absorbent member proximate the withdrawal end.

8. The feminine hygiene device of claim 7, wherein the secondary absorbent comprises polypropylene, cotton, and/or rayon and does not comprise the fluorocarbon compound.

9. The feminine hygiene device of claim 7 or 8, wherein the secondary absorbent is a color that is darker than the color of the withdrawal string.

10. The feminine hygiene device of any of claims 1-9, wherein the withdrawal string demonstrates a decreased wicking of a fluid along the withdrawal string compared to a withdrawal string not comprising the fluorocarbon compound.

## Patentansprüche

1. Damenhygienevorrichtung umfassend: einen Körper mit einen Einführende, einem Entnahmeende, einer Längsachse, wobei die Damenhygienevorrichtung einen Rückholfaden umfasst, der sich von dem Rückholende erstreckt, wobei der Rückholfaden eine oder mehrere Fluorkohlenstoffverbindungen umfasst.

2. Damenhygienevorrichtung nach Anspruch 1, wobei der Rückholfaden zwei oder mehrere Lagen Garn und/oder eine oder mehrere Gestricklagen Garn umfasst.

3. Damenhygienevorrichtung nach Anspruch 1 oder 2, wobei der Rückholfaden synthetische Fasern umfasst, und die synthetischen Fasern Polypropylen und/oder Fluorpolypropylen sind.

4. Damenhygienevorrichtung nach Anspruch 3, wobei die synthetischen Fasern gewaschene synthetische Fasern sind.

5. Damenhygienevorrichtung nach einem der Ansprüche 1 bis 4, wobei die Fluorkohlenstoffverbindung in die Fasern in Harz eingearbeitet und/oder auf die Fasern aufgebracht ist.

6. Damenhygienevorrichtung nach einem der Ansprüche 1 bis 5, wobei die fluorchemische Verbindung Polytetrafluorethylen, Perfluoralkoxy, fluoriertes Propylen, Polyvinylfluorid, Polyfluorvinyliden, ein Fluortensid oder ein Derivat ist.

7. Damenhygienevorrichtung nach einem der Ansprüche 1 bis 6, wobei die Vorrichtung ein sekundäres Absorptionsmittel umfasst, das an dem verdichteten absorbierenden Element nahe am Entnahmeende angebracht ist.

8. Damenhygienevorrichtung nach Anspruch 7, wobei das sekundäre Absorptionsmittel Polypropylen, Baumwolle und/oder Rayon umfasst und nicht die Fluorkohlenstoffverbindung umfasst.

9. Damenhygienevorrichtung nach Anspruch 7 oder 8, wobei das sekundäre Absorptionsmittel eine Farbe aufweist, die dunkler ist als die Farbe des Rückholfadens.

10. Damenhygienevorrichtung nach einem der Ansprüche 1 bis 9, wobei der Rückholfaden eine verminderte Dochtwirkung für ein Fluid entlang des Rückholfadens im Vergleich zu einem Rückholfaden aufweist, der die Fluorkohlenstoffverbindung nicht umfasst.

## Revendications

1. Dispositif hygiénique féminin comprenant : un corps possédant une extrémité d'insertion, une extrémité de retrait, un axe longitudinal, le dispositif hygiénique féminin comprenant une ficelle de retrait s'étendant à partir de l'extrémité de retrait, la ficelle de retrait comprenant un ou plusieurs composés fluorocarbure.

2. Dispositif hygiénique féminin selon la revendication 1, dans lequel la ficelle de retrait comprend deux couches ou plus de fil et/ou une ou plusieurs couches tricotées de fil.

3. Dispositif hygiénique féminin selon la revendication 1 ou 2, dans lequel la ficelle de retrait comprend des fibres synthétiques, et les fibres synthétiques sont du polypropylène et/ou du fluoropolypropylène.

4. Dispositif hygiénique féminin selon la revendication 3, dans lequel les fibres synthétiques sont des fibres synthétiques décruées.

5. Dispositif hygiénique féminin selon l'une quelconque des revendications 1 à 4, dans lequel le composé fluorocarbure est incorporé en résine dans les fibres et/ou est revêtu sur les fibres.

6. Dispositif hygiénique féminin selon l'une quelconque des revendications 1 à 5, dans lequel le composé fluorochimique est du polytétrafluoréthylène, un perfluoroalcoxy, de l'éthylène-propylène fluoré, du fluorure de polyvinyle, du polyfluorovinylidène, un agent tensioactif fluoré, ou un dérivé.

7. Dispositif hygiénique féminin selon l'une quelconque des revendications 1 à 6, où le dispositif comprend un absorbant secondaire fixé à l'élément absorbant comprimé à proximité de l'extrémité de retrait.

8. Dispositif hygiénique féminin selon la revendication 7, dans lequel l'absorbant secondaire comprend du polypropylène, du coton et/ou de la rayonne et ne comprend pas le composé fluorocarbure.

9. Dispositif hygiénique féminin selon la revendication 7 ou 8, dans lequel l'absorbant secondaire est d'une couleur qui est plus sombre que la couleur de la ficelle de retrait.

10. Dispositif hygiénique féminin selon l'une quelconque des revendications 1 à 9, dans lequel la ficelle de retrait présente une capillarité réduite d'un fluide le long de la ficelle de retrait par comparaison avec une ficelle de retrait ne comprenant pas le composé fluorocarbure.
